# EUROPÄISCHE PATENTANMELDUNG

(11) **EP 1 036 761 A1**
(43) Veröffentlichungstag der Anmeldung: **20.09.2000**
(21) Anmeldenummer: 00104238.1
(22) Anmeldetag: 01.03.2000
(51) Int. Cl.: C01B 21/22, C07C 37/60

(54) **Verfahren zur Herstellung von Lachgas**

(30) Priorität: 16.03.1999 DE 19911566
(71) Anmelder: Phenolchemie GmbH & Co. KG, 45966 Gladbeck (DE)
(72) Erfinder: Guddat, Tobias, 60489 Frankfurt (DE); Reitzmann, Andreas, 90419 Nürnberg (DE); Gerhard, Emil, Prof. Dr., 91058 Erlangen (DE)

(57) **Zusammenfassung**

Für großtechnische Anwendungen von Lachgas wie der Hydroxilierung von Benzol zu Phenol wird ein Lachgas benötigt, das möglichst kein Stickstoffmonoxid enthalten sollte.

Erfindungsgemäß kann ein Stickstoffmonoxid-freies Lachgas durch heterogen katalysierte Reduktion von Stickstoffmonoxid mit einem Reduktionsmittel, wie z.B. Wasserstoff und/oder Kohlenmonoxid gewonnen werden, wobei das Stickstoffmonoxid enthaltende Gasgemisch vorzugsweise durch Oxidation von Ammoniak erhalten wird.

## Beschreibung

Die Erfindung betrifft ein Verfahren zur Herstellung von Lachgas aus Stickstoffmonoxid sowie die bevorzugte Verwendung des erfindungsgemäß hergestellten Lachgases zur Hydroxilierung von Benzol zu Phenol. Das erfindungsgemäße Verfahren kann insbesondere auch zur Nachreinigung von Stickstoffmonoxid-haltigem Lachgas verwendet werden.

Lachgas, heute systematisch korrekt Distickstoff(mon)oxid, N₂O, genannt, ist vor allem als Inhalationsnarkotikum bekannt. Es findet jedoch auch weite technische Verbreitung, z.B. als Reinigungsmittel in der Halbleiterindustrie, als Zusatzstoff für Raketentreibstoffe oder als Treibgas für Sprays in der pharmazeutischen, der kosmetischen oder der Lebensmittelindustrie.

Eine weitere interessante Anwendung von Lachgas besteht in der katalysierten direkten Hydroxilierung von Benzol mit Lachgas zu Phenol, die erstmals von Iwamoto et al. in J. Phys. Chem., 87(6):903, 1983, beschrieben wurde. Phenol wird heute in der Hauptsache nach dem Hock-Verfahren aus Cumol in Koppelproduktion mit Aceton hergestellt. Die Phenoldirektsynthese aus Benzol und Lachgas ermöglicht hingegen prinzipiell eine von Aceton entkoppelte und unmittelbar am Weltmarkt nur für Phenol orientierte großtechnische Herstellung von Phenol Ein derartiges Verfahren zur Herstellung von Phenol beschreibt z.B. WO 95/27691.

Aus Arbeiten auf dem Gebiet der Autoabgaskatalyse ist bekannt, dass bei der katalytischen Reduktion von Stickstoffmonoxid mit Kohlenmonoxid zu Stickstoff auch Lachgas entstehen kann, das wünschenswerterweise bei der Autoabgaskatalyse mit Kohlenmonoxid zu Stickstoff und Kohlendioxid weiterreagiert (siehe z.B. S. H. Oh, Journal of Catalysis, 124, 5. 477 ff., 1990, und McCabe et al, Journal of Catalysis, 121, S, 422 ff., 1990). Diese Arbeiten zielen insgesamt darauf ab, den Ausstoß von Stickoxiden im Abgas von Automobilen durch heterogen katalysierte Reduktion mit Kohlenmonoxid zu Stickstoff und Kohlendioxid in der Gasphase zu verringern.

Rebenstorf et al. stellten in diesem Zusammenhang fest, dass bei der katalytischen Reduktion von Stickstoffmonoxid mit Kohlenmonoxid an einem bestimmten Katalysator bei niedrigen Temperaturen vermehrt Lachgas gebildet wird, während bei höheren Reaktionstemperaturen direkt eine Reduktion zu Stickstoff und Kohlendioxid erfolgt (Acta Chemica Scandinavica, A 31(1977)877-883).

Die Verwendung der katalytischen Reduktion von Stickstoffmonoxid mit Kohlenmonoxid direkt zur Herstellung von Lachgas wird in EP 0 054 965 A1 beschrieben. Es handelt sich um ein homogen katalysiertes Verfahren in flüssiger Phase. Verglichen mit früheren Laborverfahren mit Reaktion in wässriger Lösung werden durch eine Reaktionsführung in einem wasserfreien System mit vorzugsweise Methanol als Lösemittel nach eigenen Angaben "kommerziell akzeptable" Mengen an Lachgas erzielt, die jedoch heute für eine großtechnische Anwendung wie z. B. zur Phenoldirektsynthese unzureichend sind. Über die Nebenproduktbildung und die Versorgung mit Edukten sind keine bzw. keine ausreichenden Angaben gemacht. Das Verfahren ist ferner durch die Reaktionsführung in flüssiger Phase wegen der Stofftransportvorgänge relativ langsam und aufwendig. Weiterer Aufwand entsteht durch die erforderliche Katalysatorabtrennung und -rückführung.

Die Herstellung von Lachgas erfolgt daher im Labor aber auch in technischem Maßstab häufig unverändert durch thermische Zersetzung von Ammoniumnitrat, vgl. Römpp Chemie Lexikon, 9. Aufl., Bd. 5, 1992, Stichwort "Stickstoffoxide". Ein derartiges Verfahren beschreibt z.B. US 4 154 806. Die thermische Spaltung von Ammoniumnitrat ist allerdings stark exotherm und kann bei erhöhter Temperatur als Detonation verlaufen, Die Handhabung der Reaktion ist daher generell recht schwierig, wie auch US 4 154 806 belegt. Zudem wird Ammoniumnitrat vornehmlich aus Ammoniak und Salpetersäure gewonnen, wobei Salpetersäure bereits ein im Vergleich zu Ammoniak wertvollerer Ausgangsstoff ist, da Salpetersäure selbst zumeist über das Ostwald-Verfahren aus Ammoniak hergestellt wird.

US 4 102 986 beschreibt ebenfalls ein Verfahren zur Herstellung von Lachgas aus Ammoniumnitrat. Bei diesem Verfahren werden Chlorid-Ionen als Katalysator verwendet. Solche Chlorid-Ionen aufweisenden Reaktionsmischungen sind hochkorrosiv und erfordern deshalb häufig die Verwendung besonderer Werkstoffe, wie z.B. mit Titan oder Tantal plattierten Stahl, als Reaktorwerkstoffe, welche entsprechend teuer sind.

Nach EP 0 799 792 A1 ist daher wegen der preisgünstigeren Edukte die Herstellung von Lachgas in industriellem Maßstab durch katalytische Direktoxidation von Ammoniak mit Sauerstoff zu bevorzugen. EP 0 799 792 A1 beschreibt hierfür einen neuen Katalysator und ein auf diesem Katalysator basierendes Verfahren, bei dem die Reaktion von Ammoniak und Sauerstoff in Gegenwart von Wasserdampf an dem speziellen Kupfer/Manganoxid-Katalysator erfolgt. Die Katalysatorkosten und die Bildung von Stickoxiden als Nebenprodukte sollen verringert sein. Der vorgeschlagene Kupfer/Manganoxid-Katalysator muss allerdings auf sehr spezielle und vergleichsweise aufwendige Weise präpariert werden, um die gewünschten Aktivitäts- und Selektivitätseigenschaften aufzuweisen: Zunächst wird entweder durch Fällung ein Niederschlag einer Manganverbindung erzeugt, der anschließend einer wässrigen Lösung einer Kupferverbindung beigefügt wird, oder es wird umgekehrt zunächst die Kupferverbindung gefällt und der wässrigen Lösung der Manganverbindung zugegeben. Durch erneutes Fällen, Trocknen und Kälzinieren kann der gewünschte Katalysator erhalten werden. Die geringfügig stets als Nebenprodukte gebildeten Stickoxide können gemäß EP 0 799 792 A1 im Nachgang zur Reaktion durch einen Waschvorgang mit einer wässrigen, Natronlauge und Schwefelsäure enthaltenden Kaliumpermanganat-Lösung aus dem Produktgasstrom entfernt werden. Diese Nachreinigung erhöht jedoch den mit dem Verfahren verbundenen Aufwand ganz erheblich und verschlechtert zusätzlich seine Wirtschaftlichkeit.

In einigen Anwendungen von Lachgas muss nämlich ein Gehalt an Stickoxiden möglichst vermieden werden. So haben z.B. eigene Untersuchungen der Hydroxilierung von Benzol mit Lachgas zu Phenol gezeigt, dass bereits geringe Spuren von Stickstoffmonoxid im Lachgas die katalytische Phenolsynthese aus Benzol und Lachgas zum Erliegen bringen können.

Es ist daher die Aufgabe der vorliegenden Erfindung, ein möglichst einfaches und wirtschaftliches Verfahren zur Herstellung von Lachgas bereitzustellen, das neben einem hohen Potential für eine großtechnische Nutzung eine hohe Ausbeute auf der Basis herkömmlicher Katalysatoren bei möglichst geringer Nebenproduktbildung und Verunreinigung des Lachgases insbesondere durch Stickoxide, und hier speziell durch Stickstoffmonoxid, aufweist und das gleichermaßen auch auf einfache Weise zur Nachreinigung von Stickstoffmonoxid-haltigem Lachgas verwendet werden kann.

Diese Aufgabe wird erfindungsgemäß gelöst gemäß Patentanspruch 1 durch ein Verfahren zur Herstellung von Lachgas durch katalytische Reduktion von Stickstoffmonoxid mit einem Reduktionsmittel, das dadurch gekennzeichnet ist, dass die Reaktion heterogen katalysiert in der Gasphase durchgeführt wird. Vorzugsweise erfolgt die Herstellung von Lachgas derart, dass aus Ammoniak zunächst mit sauerstoffhaltigem Gas ein Lachgas und Stickstoffmonoxid enthaltendes Gasgemisch erzeugt wird, welches anschließend zur Reduktion von Stickstoffmonoxid zu Lachgas mit einem Reduktionsmittel behandelt wird. Die Oxidation des Ammoniaks kann dabei derart ausgeführt sein, dass zunächst entweder der Lachgasanteil oder der Stickstoffmonoxidanteil im Produktstrom aus der Ammoniakoxidation überwiegt.

Überraschenderweise wurde gefünden, dass sich die aus der Autoabgaskatalyse an sich bekannte katalytische Reduktion von Stickstoffmonoxid mit Kohlenmonoxid in der Gasphase zu Lachgas und Kohlendioxid technisch vorteilhaft direkt zur Herstellung von Lachgas nutzen lässt. Zur Herstellung von Lachgas war diese Reaktion bisher nur homogen katalysiert in flüssiger Phase durchgeführt worden. Bei der Umsetzung von Stickstoffmonoxid mit Kohlenmonoxid in der Gasphase konkurrieren primär die Reaktion zu Lachgas und Kohlendioxid sowie die Reaktion zu Stickstoff und Kohlendioxid miteinander. In einer Nebenreaktion kann bereits entstandenes Lachgas ferner mit Kohlenmonoxid ebenfalls zu Stickstoff und Kohlendioxid weiterreagieren.

Es wurde gefunden, dass bei einem gegebenen wirksamen Katalysator durch Einstellen einer geeigneten, in einfachen Vorversuchen leicht zu bestimmenden Reaktionstemperatur jeweils dennoch nennenswerte Ausbeuten an Lachgas erhalten werden können. Das Molverhältnis von Stickstoffmonoxid zu Kohlenmonoxid im Eduktstrom sollte dabei vorzugsweise kleiner oder gleich 2 sein. Durch eine geeignete Wahl des Katalysators und der entsprechenden Reaktionsbedingungen lassen sich sogar eine vollständige Umsetzung von Stickstoffmonoxid sowie Lachgasausbeuten von über 90 % bezogen auf eingesetztes Stickstoffmonoxid erreichen.

Es wurde überraschenderweise gefunden, dass die erfindungsgemäße katalytische Reduktion von Stickstoffmonoxid auch mit anderen Reduktionsmitteln, insbesondere mit Wasserstoff, Wasserstoff aufweisenden Gemischen, wie z.B. Synthesegas, welches Wasserstoff und Kohlenmonoxid aufweist, heterogen katalysiert in der Gasphase durchgeführt werden kann. Auch bei der Verwendung von anderen Reduktionsmitteln, wie z.B. Wasserstoff oder Wasserstoff aufweisenden Gemischen, wurde gefunden, dass bei einem gegebenen wirksamen Katalysator durch Einstellen einer geeigneten, in einfachen Vorversuchen leicht zu bestimmenden Reaktionstemperatur jeweils dennoch nennenswerte Ausbeuten an Lachgas erhalten werden können. Das Molverhältnis von Stickstoffmonoxid zu Reduktionsmittel im Eduktstrom sollte dabei vorzugsweise kleiner oder gleich 2 sein. Durch eine geeignete Wahl des Katalysators und der entsprechenden Reaktionsbedingungen lassen sich sogar eine vollständige Umsetzung von Stickstoffmonoxid sowie Lachgasausbeuten von über 90 % bezogen auf eingesetztes Stickstoffmonoxid erreichen.

Bei der Umsetzung von Lachgas mit Wasserstoff aufweisenden Gemischen wurde außerdem gefunden, dass geringe Mengen Kohlenmonoxid die Reaktion von Stickstoffmonoxid zu Lachgas begünstigen, obwohl das Kohlenmonoxid kaum reagiert. Außerdem wurde bei diesen Reaktionsbedingungen keine Desaktivierung des verwendeten Katalysators (durch das Kohlenmonoxid) während der Versuchsdauer festgestellt. Der Grund für dieses Verhalten ist noch ungeklärt.

Überraschenderweise lässt sich daher mit Hilfe des erfindungsgemäßen Verfahrens auf sehr einfache und wirtschaftliche Weise Lachgas herstellen, das aufgrund der Reaktion des Stickstoffmonoxids mit Kohlenmonoxid ohne spezielle Nachreinigung einen sehr geringen Gehalt an Stickoxiden, insbesondere jedoch an Stickstoffmonoxid, aufweist. Vorzugsweise wird das erfindungsgemäße Verfahren so betrieben, dass im Eduktstrom enthaltenes Stickstoffmonoxid vollständig umgesetzt wird und damit im Produktgas nicht mehr enthalten ist. Ein auf diese Weise erfindungsgemäß hergestelltes Lachgas ist damit insbesondere vorteilhaft für die Direktsynthese von Phenol einsetzbar, weil es ohne zusätzliche aufwendige Reinigung des Lachgases zur Verringerung des Stickstoffmonoxidgehalts und damit ohne Beeinträchtigung der Wirtschaftlichkeit der Phenoldirektsynthese verwendet werden kann. Auch bei der Verwendung von anderen Reduktionsmitteln wie z.B. Wasserstoff oder Wasserstoff aufweisenden Gemischen wird ein Lachgas erhalten, das geringe Konzentrationen an Stickstoffmonoxid aufweist. Bei der Verwendung von Wasserstoff oder Wasserstoff aufweisenden Gemischen als Reduktionsmittel entsteht jedoch aus dem Wasserstoff bei der Reduktion des Stickstoffmonoxids zum Lachgas Wasser. Da dieses Wasser z.B. bei der Verwendung des Lachgases bei der Direktsynthese von Phenol, störend wirken kann, kann je nach Verwendungszweck des erfindungsgemäße hergestellten Lachgases, entstandenes Wasser auf dem Fachmann bekannte Weise, z.B. durch Auskondensieren oder Adsorption des Wassers an Molekularsieben, Silicagelen oder ähnlichen zur Gastrocknung geeigneten Verbindungen, aus dem Lachgas entfernt werden.

Der vorzugsweise als Ausgangsstoff zur Herstellung von Lachgas im Rahmen des erfindungsgemäßen Verfahrens verwendete Ammoniak wird industriell weltweit in großen Mengen und damit preisgünstig zumeist nach dem Haber-Bosch-Verfahren aus Stickstoff und Wasserstoff erzeugt und besitzt als wichtiger Grundstoff für die chemische Industrie eine hohe Verfügbarkeit.

Die Oxidation von Ammoniak zu überwiegend Stickstoffmonoxid ist aus dem Ostwald-Verfahren zur industriellen Herstellung von Salpetersäure auch im großtechnischen Maßstab bestens bekannt. Nach Ullmann's encyclopedia of industrial chemistry, Band A17, S. 293 ff., 1991, ist diese Oxidation von Ammoniak eine der effektivsten katalytischen Reaktionen, in der in technischem Maßstab Ausbeuten an Stickstoffmonoxid von bis zu 98 % bezogen auf Ammoniak erreicht werden. Als Reaktionspartner dient sauerstoffhaltiges Gas, vorzugsweise Luft, aber auch reiner Sauerstoff oder ein Gemisch aus beidem, so dass neben Stickstoffmonoxid Wasser entsteht. Der Ammoniakanteil bei Ammoniak/Luft-Eduktgemischen wird in der Regel durch die untere Explosionsgrenze vorgegeben und beträgt je nach den Druckverhältnissen bis zu 13,5 Gew.-%. Die Oxidationsreaktion wird üblicherweise bei Drücken bis zu 12 bar absolut und Temperaturen zwischen ca. 840 °C und 950 °C durchgeführt Als Katalysatoren haben sich insbesondere Platinnetze, die zur Verbesserung der Katalysatoreigenschaften einen Anteil von 5 bis 10 Gew.-% Rhodium aufweisen können, bewährt. Zur Durchführung der Reaktion sind verschiedene Reaktortypen bzw. -systeme bekannt, vgl. z.B. Ullmann's encyclopedia of industrial chemistry, Band A17, S. 293 ff., 1991. Bevorzugt werden für die Ammoniakoxidation Reaktoren mit Wärmerückgewinnungssystemen zur Rückgewinnung der freiwerdenen Reaktionsenergie verwendet.

Als Nebenprodukte entstehen unter den genannten Reaktionsbedingungen bei dieser Form der Ammoniakoxidation in geringem Umfang Stickstoff und wie erwähnt bereits das erfindungsgemäß herzustellende Lachgas. Der Produktgasstrom der Ammoniakoxidation kann daher bevorzugt unmittelbar der Reduktion des Stickstoffmonoxids zu Lachgas zugeführt werden, da in den meisten Anwendungsfällen für Lachgas ein Gehalt an Stickstoff und Wasser nicht störend ist. In den Fällen, in denen das erfindungsgemäß hergestellte Lachgas später in Anwendungen eingesetzt wird, die kritisch in Bezug auf einen Gehalt an Stickstoff und/oder Wasser sind, kann der Produktgasstrom aus der Ammoniakoxidation zu Stickstoffmonoxid allerdings auch zunächst einem oder mehreren Reinigungsschritten unterzogen werden. Soll das gebildete Wasser abgetrennt werden, so kann eine sehr einfache Trocknung des Gasstroms auf dem Fachmann bekannte Weise beispielsweise durch Auskondensieren oder Adsorption des Wassers an Molekularsieben oder Silicagelen erfolgen. Für eine spätere Verwendung des erfindungsgemäß hergestellten Lachgases zur Phenoldirektsynthese ist ein derartiger Trocknungsschritt bei der Lachgasherstellung bevorzugt, um eine Beeinflussung der Hydroxilierung von Benzol sicher auszuschließen, wobei der Trocknungsschritt vor und/oder nach der Reduktion des Stickstoffmonoxids erfolgen kann. Für den Fall, dass das bei der Ammoniakoxidation entstandene Wasser negativen Einfluss auf die Katalysatoreigenschaften des zur Reduktion von Stickstoffmonoxid mit Kohlenmonoxid verwendeten Katalysators besitzt, ist ein Trocknungsschritt vor der Stickstoffmonoxidreduktion bevorzugt. Ein Gehalt an Stickstoff ist dagegen für die Stickstoffmonoxidreduktion und die meisten bekannten Lachgasanwendungen unkritisch. Der Stickstoffgehalt kann aber bei Bedarf etwa durch Verflüssigung, Rektifikation bei hohem Druck oder durch geeignete Membrantrennverfahren zur Abtrennung von N₂ verringert werden.

Bei ansonsten analogen Bedingungen bei der Ammoniakoxidation lässt sich durch niedrigere Reaktionstemperaturen von 250 °C bis 450 °C und vorzugsweise einen entsprechenden speziellen Katalysator wie in EP 0 799 792 A1 anstelle von Platinnetzen die Produktausbeute bei der Ammoniakoxidation auch direkt hin zu Lachgas und Stickstoff verschieben. Es werden stets auch Wasser und Stickstoffmonoxid gebildet, die beide in Lachgasanwendungen wie der Phenoldirektsynthese auch nur in geringen Mengen unerwünscht sind und daher jeweils durch geeignete Reinigungsprozesse entfernt werden müssen. Der Gehalt an Stickstoffmonoxid kann durch die erfindungsgemäße heterogen katalysierte Reduktion von Stickstoffmonoxid mit einem Reduktionsmittel in der Gasphase verringert oder beseitigt werden.

Der Wassergehalt und bei Bedarf auch der Stickstoffgehalt können durch die bereits genannten Verfahren verringert werden, wobei die Reihenfolge der verschiedenen Reinigungsschritte - wie weiter oben bereits gezeigt - variieren kann. Das erfindungsgemäße Verfahren dient in diesem Zusammenhang quasi zur Nachreinigung eines Stickstoffmonoxid enthaltenden Lachgases. Es bietet dabei den Vorteil, dass Stickstoffmonoxid nicht nur auf - verglichen mit einer Wäsche mit einer Kaliumpermanganatlösung - einfache Weise entfernt, sondern sogar ohne Ausbeuteverlust in das gewünschte Wertprodukt Lachgas überführt wird.

Welche Verfahrensvariante bei der bevorzugten Ammoniakoxidation als erstem Verfahrensschritt zur Herstellung von Lachgas zu bevorzugen ist, richtet sich vorzugsweise nach wirtschaftlichen Gesichtspunkten. Vor diesem Hintergrund ist die Ammoniakoxidation direkt zu überwiegend Lachgas zu bevorzugen, weil sie den geringeren Bedarf an Reduktionsmittel als Reaktanden aufweist. Steht das Reduktionsmittel allerdings zu vernachlässigbaren Kosten zur Verwertung zur Verfügung, bietet die Route über Stickstoffmonoxid Vorteile, da sie einfach umsetzbar ist und eine geringere Nebenproduktbildung in Bezug auf andere Stickoxide und Stickstoff aufweist. Erfindungsgemäß kann aber in jedem Fall ein Gehalt von Stickstoffmonoxid im Lachgas durch vollständige Reduktion des bei der Ammoniakoxidation entstandenen Stickstoffmonoxids zu Lachgas vermieden werden.

Die heterogen katalysierte Umsetzung von Stickstoffmonoxid mit einem Reduktionsmittel in der Gasphase erfolgt erfindungsgemäß in Abhängigkeit vom verwendeten Katalysator bei Temperaturen von über 40 °C und Drücken von 1 bis 20 bar absolut. Vorzugsweise wird als Reduktionsmittel Kohlenmonoxid und/oder Wasserstoff oder Synthesegas verwendet.

Als Katalysatoren können beispielsweise herkömmliche Typen wie Platin/Aluminiumoxid(Al₂O₃)-Katalysatoren, Rhodium/Al₂O₃-Katalysatoren, Palladium/Al₂O₃-Katalysatoren, Ruthenium/Al₂O₃-Katalysatoren, Lanthan-Eisen- Zeolithkatalysatoren oder Chrom(II)/Silicagel(SiO₂)-Katalysatoren verwendet werden. Geeignete Katalysatoren sind auch edelmetallhaltige, also z.B. Platin-, Rhodium- oder Palladiumhaltige, oder Chrom(II)haltige Zeolithe. Neben Silicagel und γ-Aluminiumoxid sind also auch Zeolithe, insbesondere vom Typ ZSM-5, als Trägermaterial geeignet. Ebenfalls als Trägermaterialien geeignet sind Träger, die TiO₂, ZrO₂ oder Aktivkohle aufweisen.

Geeignete Reaktionstemperaturen für ausgewählte Katalysatoren und Reduktionsmittel können wie erwähnt in einfachen Laborversuchen ermittelt werden, in denen die Reaktion in Abhängigkeit von der Temperatur durchgeführt und jeweils die Lachgasausbeute ermittelt wird.

Bevorzugt beträgt bei der Verwendung von Kohlenmonoxid bzw. überwiegend Kohlenmonoxid aufweisenden Gemischen als Reduktionsmittel die Reaktionstemperatur 200 °C bis 450 °C bei einem Reaktionsdruck von 1 bis 10 bar absolut. Als Katalysatoren werden bei diesen Reaktionsbedingungen besonders bevorzugt Platin/Aluminiumoxid(Al₂O₃)-Katalysatoren mit Platingehalten von 0,01 bis 5 Gew.-%, Rhodium/Al₂O₃ -Katalysatoren mit Rhodiumgehalten von bis zu 5 Gew.-%, Lanthan-Eisen- Zeolithkatalysatoren oder Chrom(II)/Silicagel(SiO₂)-Katalysatoren verwendet werden. Ganz besonders bevorzugt sind Rh/Al₂O₃-Katalysatoren mit 1 bis 5 Gew.-% Rhodium auf einem γ-Aluminiumoxidträger sowie Cr/SiO₂-Katalysatoren mit 1 bis 5 Gew. -% Chrom(II)oxid.

Bei der Verwendung von Wasserstoff bzw. überwiegend Wasserstoff aufweisenden Gemischen, wie z.B. Synthesegas, welches Wasserstoff und Kohlenmonoxid, z.B. in einem molaren Verhältnis von 3 zu 1 aufweist, als Reduktionsmittel beträgt die Reaktionstemperatur vorzugsweise von 50 °C bis 350 °C bei einem Reaktionsdruck von 1 bis 10 bar absolut. Als Katalysatoren werden bei diesen Reaktionsbedingungen besonders bevorzugt Palladium/Aluminiumoxid(Al₂O₃)-Katalysatoren mit Palladiumgehalten von 0,01 bis 5 Gew.-% oder Platin/Al₂O₃ -Katalysatoren mit Platingehalten von bis zu 5 Gew. -% verwendet. Ganz besonders bevorzugt ist ein Pt/Al₂O₃-Katalysator mit einem Platingehalt von 1 bis 5 Gew.-% und/oder ein Pd/Al₂O₃-Katalysator mit 1 bis 5 Gew.-% Palladium auf einem γ-Aluminiumoxidträger.

Als Reaktoren sind, unabhängig von der Wahl des Reduktionsmittels, herkömmliche gekühlte Festbettreaktoren geeignet.

Es wurde ferner gefunden, dass je nach verwendetem Katalysator und verwendetem Reduktionsmittel die Zusammensetzung des Produktgasstroms wesentlich von den Konzentrationsverhältnissen im Eduktgasstrom beeinflusst werden kann. Ist die molare Stickstoffmonoxidkonzentration im Eduktstrom größer als die molare Konzentration an Kohlenmonoxid, wird bei Verwendung eines Chrom(II)-Katalysators, insbesondere bei der Verwendung eines Chrom(II)/Silicagel-Katalysators die Umsetzung von Stickstoffmonoxid und Kohlenmonoxid zu Lachgas und Kohlendioxid gegenüber der Reaktion zu Stickstoff und Kohlendioxid bevorzugt, allerdings auf Kosten eines unvollständigen Umsatzes von Stickstoffmonoxid. Erfindungsgemäß wird daher ein Molverhältnis von Stickstoffmonoxid zu Kohlenmonoxid von kleiner oder gleich 1 im Eduktgasgemisch bevorzugt. Bei der Verwendung edelmetallhaltiger Katalysatoren wurde dieser Zusammenhang nicht gefunden, weshalb bei der Verwendung dieser Katalysatoren vorzugsweise ein Molverhältnis von Stickstoffmonoxid zu Kohlenmonoxid von kleiner gleich 2 eingestellt wird. Bei der Verwendung von Wasserstoff oder überwiegend Wasserstoff aufweisenden Gemischen als Reduktionsmittel, wie z.B. Synthesegas, wurde dieser Zusammenhang ebenfalls nicht festgestellt. Erfindungsgemäß wird deshalb ein Molverhältnis von Stickstoffmonoxid zu Wasserstoff bzw. Reduktionsmittel (Also z.B. Wasserstoff und Kohlenmonoxid) von kleiner gleich 2 im Eduktgasgemisch bevorzugt. Auf diese Weise kann - zumindest an den bevorzugt eingesetzten Katalysatoren - ein vollständiger Umsatz des Stickstoffmonoxids bei Lachgasausbeuten von über 90 % erzielt werden, wodurch das gewonnene Lachgas frei von Stickstoffmonoxid ist. Es ist vorteilhaft, die optimale Zusammensetzung des Eduktgasstroms in entsprechenden einfachen Vorversuchen in Abhängigkeit vom Katalysator zu bestimmen. So wurden bei der Verwendung von Palladium oder Rhodium aufweisenden Katalysatoren gute Lachgasausbeuten bei vollständigem Umsatz des Stickstoffmonoxids mit molaren Verhältnissen von Stickstoffmonoxid zu Reduktionsmittel von 10 : 7 oder 4 : 3 erzielt.

Wird Lachgas gemäß der bevorzugten Ausführung des erfindungsgemäßen Verfahrens durch Oxidation von Ammoniak und anschließende vollständige Reduktion von entstandenem Stickstoffmonoxid hergestellt, so weist das erhaltene Lachgas keinerlei Stickstoffmonoxid und nur in geringem Umfang andere Stickoxide auf so dass es sich - zumindest nach Trocknung - vorzüglich zur Phenoldirektsynthese eignet.

Der Gehalt an Kohlendioxid, das beim erfindungsgemäßen Verfahren gebildet werden kann, im Produktgasstrom kann bei Bedarf - der in vielen Lachgasanwendungen nicht vorliegt - beispielsweise durch Waschen mit alkalischer Lösung verringert oder beseitigt werden. Der Gehalt des als Nebenprodukt entstandenen Stickstoffs lässt sich gegebenenfalls z.B. durch Rektifikation bei hohem Druck, durch Verflüssigung oder durch geeignete Membrantrennverfahren zur Abtrennung von N₂ senken.

Das zur erfindungsgemäßen Reduktion von Stickstoffmonoxid als Reduktionsmittel benötigte Kohlenmonoxid kann großtechnisch z.B. durch Steam-Reforming von Erdgas und Naphta, aus partieller Oxidation von schwerem Heizöl oder durch Kohlevergasung erhalten werden. Hierbei wird ein so genanntes Synthesegas aus Kohlenmonoxid und Wasserstoff erhalten, aus dem sich Kohlenmonoxid beispielsweise durch reversible Komplexbildung mit Kupfer(I)-Salzlösungen oder durch Tieftemperaturabtrennung, die sich die unterschiedlichen Siedepunkte von Wasserstoff und Kohlenmonoxid zu nutze macht, abtrennen lässt. Kohlenmonoxid ist damit mittels bekannter Verfahren in großen Mengen verfügbar.

Auf diese Weise ist aus Synthesegas auch der Wasserstoff, der ebenfalls als Reduktionsmittel eingesetzt werden kann, verfügbar.

Vorzugsweise wird das Synthesegas direkt als Reduktionsmittel verwendet, da Synthesegas leichter (billiger) verfügbar ist. Da bei der Verwendung von Synthesegas bei der Reduktion mit einem geeigneten Katalysator die Reaktionstemperatur geringer gehalten werden kann, ergibt sich auch noch ein weiterer Vorteil, es fallen nämlich geringere Energiekosten an als bei der Verwendung von Kohlenmonoxid als Reduktionsmittel.

Das erfindungsgemäße Verfahren bietet damit insbesondere in seiner Kombination mit der Ammoniakoxidation wegen der einfachen und preisgünstigen Verfügbarkeit der Ausgangsstoffe ein hohes und wirtschaftliches Potential zur Herstellung von Lachgas, das frei von Stickstoffmonoxid ist und je nach Verfahrensweise nur in geringem Maße andere Stickoxide aufweist.

Nach bisher bekannten Verfahren hergestelltes Lachgas wurde z.B. als Anästhetikum eingesetzt und hatte damit ganz anderen Reinheitsanforderungen zu genügen. Der Einsatz des nach dem hier vorgeschlagenen Verfahren hergestellten Distickstoffmonoxids als Oxidationsmittel in der Benzolhydroxilierung ist u.a. deshalb wirtschaftlich, da hier z.B. Kohlenmonoxid- und Kohlendioxid-Verunreinigungen erlaubt, teilweise sogar vorteilhaft sein können.

Das erfindungsgemäße Verfahren wird durch die nachfolgenden Beispiele näher erläutert, ohne darauf beschränkt zu sein:

### Beispiel 1:

In einem thermostatisierten Festbettrohrreaktor mit einem Innendurchmesser von 7 mm, einer Rohrlänge von 190 mm und einer Katalysatorschütthöhe von 50 mm (wobei der Katalysator insgesamt in der Nähe des Eintritts in den Reaktor angeordnet ist) werden Stickstoffmonoxid und Kohlenmonoxid kontinuierlich zur Reaktion gebracht. Stickstoffmonoxid und Kohlenmonoxid werden aus getrennten Vorlagen dem Reaktor derart zugeführt, dass das Molverhältnis von Stickstoffmonoxid zu Kohlenmonoxid im Eduktgasstrom 1,0 beträgt. Als Katalysator kommt ein Rh/Al₂O₃ -Katalysator mit einem Rhodiumgehalt von 5 Gew.-% zum Einsatz, wobei der γ-Aluminiumoxidträger eine BET-Oberfläche von 309 m²/g und ein Porenvolumen von 0,28 ml/g aufweist. Die Reaktionstemperatur beträgt 220 °C bei einem Reaktionsdruck von 1 bar absolut.

Am Reaktorausgang werden die Gase Stickstoff, Stickstoffmonoxid, Lachgas, Kohlenmonoxid und Kohlendioxid mit Hilfe eines Gaschromatographen (Fa. Perkin-Elmer, Typ 8700 ht) und eines Chemilumineszenzdetektors (Fa. TECAN, Typ CLD 7000 EL) detektiert. Unter den gewählten Reaktionsbedingungen kann kein Stickstoffmonoxid mehr im Produktgasstrom nachgewiesen werden, d.h. der Stickstoffmonoxid-Umsatz beträgt 100 %. Ferner wird die Ausbeute an Lachgas zu ca. 75 % bezogen auf Stickstoffmonoxid bestimmt.

### Beispiel 2:

In der Versuchsapparatur aus Beispiel 1 wird Stickstoffmonoxid mit Kohlenmonoxid bei ca. 270 °C und einem Reaktionsdruck von 1 bar absolut umgesetzt. Das Molverhältnis der Edukte Stickstoffmonoxid zu Kohlenmonoxid beträgt 0,67. Als Katalysator dient ein Cr/SiO₂-Katalysator mit einem Chrom(II)-Oxidgehalt von 1 Gew.-%. Das zur Katalysatorpräparation verwendete Silicagel besitzt einen mittleren Porendurchmesser von 100 Angström, einen Partikeldurchmesser von 50 µm, eine Oberfläche von 520 m²/g und ein Porenvolumen von 0,93 ml/g.

Wieder werden am Reaktorausgang die Gase Stickstoff, Stickstoffmonoxid, Lachgas, Kohlenmonoxid und Kohlendioxid mit Hilfe eines Gaschromatographen (Fa. Perkin-Elmer, Typ 8700 ht) und eines Chemilumineszenzdetektors (Fa. TECAN, Typ CLD 7000 EL) detektiert. Unter den gewählten Reaktionsbedingungen kann kein Stickstoffmonoxid mehr im Produktgasstrom nachgewiesen werden, d.h. der Stickstoffmonoxid-Umsatz beträgt 100 %. Ferner wird die Ausbeute an Lachgas zu ca. 95 % bezogen auf Stickstoffmonoxid bestimmt.

### Beispiel 3:

In der Versuchsapparatur aus Beispiel 1 wird Stickstoffmonoxid mit Kohlenmonoxid bei ca. 430 °C und einem Reaktionsdruck von 1 bar absolut umgesetzt. Das Molverhältnis der Edukte Stickstoffmonoxid zu Kohlenmonoxid beträgt 1,0. Als Katalysator dient ein Pt/Al₂O₃-Katalysator mit einem Platingehalt von 0,5 Gew.-%. Der zur Katalysatorpräparation verwendete γ-Al₂O₃-Träger besitzt eine BET-Oberfläche von 278 m²/g und ein Porenvolumen von 0,24 ml/g.

Wieder werden am Reaktorausgang die Gase Stickstoff, Stickstoffmonoxid, Lachgas, Kohlenmonoxid und Kohlendioxid mit Hilfe eines Gaschromatographen (Fa. Perkin-Elmer, Typ 8700 ht) und eines Chemilumineszenzdetektors (Fa. TECAN, Typ CLD 7000 EL) detektiert. Unter den gewählten Reaktionsbedingungen kann kein Stickstoffmonoxid mehr im Produktgasstrom nachgewiesen werden, d.h. der Stickstoffmonoxid-Umsatz beträgt 100 %. Ferner wird die Ausbeute an Lachgas zu knapp 40 % bezogen auf Stickstoffmonoxid bestimmt.

### Beispiel 4:

In der Versuchsapparatur aus Beispiel 1 wird Stickstoffmonoxid mit Kohlenmonoxid bei ca. 250 °C und einem Reaktionsdruck von 1 bar absolut umgesetzt. Das Molverhältnis der Edukte Stickstoffmonoxid zu Kohlenmonoxid beträgt 1,0. Als Katalysator dient ein Ru/Al₂O₃-Katalysator mit einem Rutheniumgehalt von 5 Gew.-%. Der zur Katalysatorpräparation verwendete γ-Al₂O₃-Träger besitzt eine BET-Oberfläche von 278 m²/g und ein Porenvolumen von 0,24 ml/g.

Wieder werden am Reaktorausgang die Gase Stickstoff, Stickstoffmonoxid, Lachgas, Kohlenmonoxid und Kohlendioxid mit Hilfe eines Gaschromatographen (Fa. Perkin-Elmer, Typ 8700 ht) und eines Chemilumineszenzdetektors (Fa. TECAN, Typ CLD 7000 EL) detektiert. Unter den gewählten Reaktionsbedingungen konnten nur geringe Mengen an Stickstoffmonoxid im Produktgasstrom nachgewiesen werden, d. h. der Stickstoffmonoxid-Umsatz beträgt nicht ganz 100 %. Ferner wird die Ausbeute an Lachgas zu knapp 70 % bezogen auf Stickstoffmonoxid bestimmt.

### Beispiel 5:

In der Versuchsapparatur aus Beispiel 1 wird Stickstoffmonoxid mit Wasserstoff bei ca. 100 °C und einem Reaktionsdruck von 1 bar absolut umgesetzt. Das Molverhältnis der Edukte Stickstoffmonoxid zu Wasserstoff beträgt 10 : 7. Als Katalysator dient ein Pd/Al₂O₃-Katalysator mit einem Palladiumgehalt von 5 Gew.-%. Der zur Katalysatorpräparation verwendete γ-Al₂O₃-Träger besitzt eine BET-Oberfläche von 278 m²/g und ein Porenvolumen von 0,24 ml/g.

Wieder werden am Reaktorausgang die Gase Stickstoff, Stickstoffmonoxid und Lachgas mit Hilfe eines Gaschromatographen (Fa. Perkin-Elmer, Typ 8700 ht) und eines Chemilumineszenzdetektors (Fa. TECAN, Typ CLD 7000 EL) detektiert. Unter den gewählten Reaktionsbedingungen kann kein Stickstoffmonoxid mehr im Produktgasstrom nachgewiesen werden, d.h. der Stickstoffmonoxid-Umsatz beträgt 100 %. Ferner wird die Ausbeute an Lachgas zu knapp 84 % bezogen auf Stickstoffmonoxid bestimmt.

Beim Überprüfen der optimalen Reaktionstemperatur wurde festgestellt, dass bei den genannten Reaktionsbedingungen zwei Ausbeutemaxima bei Temperaturen von 100 °C und 160 °C vorhanden sind, wobei das Ausbeutemaximum bei 100 °C sehr scharf ist, dass heißt auf einen engen Temperaturbereich begrenzt, während das zweite Maximum bei 160 °C breiter ist, dass heißt nicht auf einen so engen Temperaturbereich begrenzt wie das Ausbeutemaximum bei 100 °C. Bei der Durchführung der Reaktion bei 160 °C konnte auch nach einer Versuchsdauer von 70 Stunden keine Desaktivierung des Katalysators festgestellt werden.

### Beispiel 6:

In der Versuchsapparatur aus Beispiel 1 wird Stickstoffmonoxid mit Synthesegas, welches ein Molverhältnis von Wasserstoff zu Kohlenmonoxid von 3:1 aufweist, bei ca. 130 °C und einem Reaktionsdruck von 1 bar absolut umgesetzt. Das Molverhältnis der Edukte Stickstoffmonoxid zu Synthesegas beträgt 3 : 2. Als Katalysator dient ein Pd/Al₂O₃-Katalysator mit einem Palladiumgehalt von 5 Gew.-%. Der zur Katalysatorpräparation verwendete γ-Al₂O₃-Träger besitzt eine BET-Oberfläche von 278 m²/g und ein Porenvolumen von 0,24 ml/g.

Wieder werden am Reaktorausgang die Gase Stickstoff, Stickstoffmonoxid und Lachgas mit Hilfe eines Gaschromatographen (Fa. Perkin-Elmer, Typ 8700 ht) und eines Chemilumineszenzdetektors (Fa. TECAN, Typ CLD 7000 EL) detektiert. Unter den gewählten Reaktionsbedingungen kann kein Stickstoffmonoxid mehr im Produktgasstrom nachgewiesen werden, d.h. der Stickstoffmonoxid-Umsatz beträgt 100 %. Ferner wird die Ausbeute an Lachgas zu knapp 95 % bezogen auf Stickstoffmonoxid bestimmt.

Auch nach einer Versuchsdauer von 80 Stunden konnte keine Desaktivierung des Katalysators festgestellt werden.

### Beispiel 7:

In der Versuchsapparatur aus Beispiel 1 wird Stickstoffmonoxid mit Synthesegas, welches ein Molverhältnis von Wasserstoff zu Kohlenmonoxid von 3 : 1 aufweist, bei ca. 130 °C und einem Reaktionsdruck von 1 bar absolut umgesetzt. Das Molverhältnis der Edukte Stickstoffmonoxid zu Synthesegas beträgt 3 : 2. Als Katalysator dient ein Pt/Al₂O₃-Katalysator mit einem Platingehalt von 5 Gew.-%. Der zur Katalysatorpräparation verwendete γ-Al₂O₃-Träger besitzt eine BET-Oberfläche von 278 m²/g und ein Porenvolumen von 0,24 ml/g.

Wieder werden am Reaktorausgang die Gase Stickstoff, Stickstoffmonoxid und Lachgas mit Hilfe eines Gaschromatographen (Fa. Perkin-Elmer, Typ 8700 ht) und

eines Chemilumineszenzdetektors (Fa. TECAN, Typ CLD 7000 EL) detektiert. Unter den gewählten Reaktionsbedingungen kann kein Stickstoffmonoxid mehr im Produktgasstrom nachgewiesen werden, d.h. der Stickstoffmonoxid-Umsatz beträgt 100 %. Ferner wird die Ausbeute an Lachgas zu knapp 80 % bezogen auf Stickstoffmonoxid bestimmt.

Auch nach einer Versuchsdauer von 24 Stunden konnte keine Desaktivierung des Katalysators festgestellt werden.

## Patentansprüche

1. Verfahren zur Herstellung von Lachgas durch katalytische Reduktion von Stickstoffmonoxid mit einem Reduktionsmittel,
dadurch gekennzeichnet,
dass die Reaktion heterogen katalysiert in der Gasphase durchgeführt wird.

2. Verfahren nach Anspruch 1,
dadurch gekennzeichnet,
dass die Reaktion bei Temperaturen über 40 °C und Drücken von 1 bis 20 bar absolut erfolgt.

3. Verfahren nach einem der Ansprüche 1 oder 2,
dadurch gekennzeichnet,
dass das Stickstoffmonoxid bei der Oxidation von Ammoniak mit sauerstoffhaltigem Gas erzeugt wird.

4. Verfahren nach Anspruch 3,
dadurch gekennzeichnet,
dass die Ammoniakoxidation durch ein Ostwald-Verfahren erfolgt.

5. Verfahren nach zumindest einem der vorherigen Ansprüche,
dadurch gekennzeichnet,
dass der Stickstoffmonoxid enthaltende Eduktgasstrom vor der Reduktion zu Lachgas getrocknet wird.

6. Verfahren nach zumindest einem der vorherigen Ansprüche,
dadurch gekennzeichnet,
dass der aus Stickstoffmonoxid hergestelltes Lachgas enthaltende Produktgasstrom zur Verringerung des Wassergehalts getrocknet wird.

7. Verfahren nach zumindest einem der vorherigen Ansprüche,
dadurch gekennzeichnet,
dass als Katalysatoren Palladium/Al₂O₃-Katalysatoren, Platin/Al₂O₃-Katalysatoren, Rhodium/Al₂O₃-Katalysatoren, Ruthenium/Al₂O₃-Katalysatoren, Lanthan-Eisen-Zeolithkatalysatoren und/oder Chrom(II)/SiO₂-Katalysatoren verwendet werden.

8. Verfahren nach zumindest einem der vorherigen Ansprüche,
dadurch gekennzeichnet,
dass als Reduktionsmittel Kohlenmonoxid und/oder Wasserstoff verwendet werden.

9. Verfahren nach Anspruch 8
dadurch gekennzeichnet,
dass als Reduktionsmittel Kohlenmonoxid verwendet wird.

10. Verfahren nach Anspruch 9,
dadurch gekennzeichnet,
dass die Reaktionstemperatur 200 bis 450 °C beträgt.

11. Verfahren nach einem der Ansprüche 9 oder 10,
dadurch gekennzeichnet,
dass der Katalysator ein Rh/Al₂O₃-Katalysator mit einem Rhodiumgehalt von 1 bis 5 Gew.-% oder ein Cr/SiO₂-Katalysator mit einem Chrom(II)-Oxidgehalt von 1 bis 5 Gew.-%, bevorzugt 1 Gew.-%, ist.

12. Verfahren nach zumindest einem der Ansprüche 9 bis 11,
dadurch gekennzeichnet,
dass das Molverhältnis von Stickstoffmonoxid zu Kohlenmonoxid im Eduktgasstrom kleiner oder gleich 2 ist.

13. Verfahren nach Anspruch 8
dadurch gekennzeichnet,
dass als Reduktionsmittel Wasserstoff verwendet wird.

14. Verfahren nach Anspruch 8
dadurch gekennzeichnet,
dass als Reduktionsmittel Wasserstoff und Kohlenmonoxid aufweisendes Synthesegas verwendet wird.

15. Verfahren nach zumindest einem der Ansprüche 13 oder 14,
dadurch gekennzeichnet,
dass die Reaktionstemperatur 50 bis 350 °C beträgt.

16. Verfahren nach einem der Ansprüche 13 bis 15,
dadurch gekennzeichnet,
dass der Katalysator ein Pt/Al₂O₃-Katalysator mit einem Platingehalt von 1 bis 5 Gew.-% und/oder ein Pd/Al₂O₃-Katalysator mit einem Palladiumgehalt von 1 bis 5 Gew. -%, bevorzugt 1 Gew.-%, ist.

17. Verfahren nach zumindest einem der Ansprüche 13 bis 16,
dadurch gekennzeichnet,
dass das Molverhältnis von Stickstoffmonoxid zu Reduktionsmittel im Eduktgasstrom kleiner oder gleich 2 ist.

18. Verfahren nach zumindest einem der Ansprüche 1 bis 17 zur Verringerung des Stickstoffmonoxidgehalts in Lachgas.

19. Verwendung des nach zumindest einem der Ansprüche 1 bis 18 hergestellten Lachgases zur Hydroxilierung von Benzol zu Phenol.
